# EUROPÄISCHE PATENTSCHRIFT

(11) **EP 3 260 999 B1**
(45) Veröffentlichungstag und Bekanntmachung des Hinweises auf die Patenterteilung: **04.08.2021**
(21) Anmeldenummer: 16176234.9
(22) Anmeldetag: 24.06.2016
(51) Int. Cl.: G06F 30/20

(54) **SYSTEM ZUM SIMULIEREN VON SENSOREN**
SYSTEM FOR SIMULATING SENSORS
SYSTEME DE SIMULATION DE CAPTEURS

(43) Veröffentlichungstag der Anmeldung: 27.12.2017
(73) Patentinhaber: SICK AG, 79183 Waldkirch (DE)
(72) Erfinder: Bornstein, Patrick, 79117 Freiburg (DE); Alt, Gerhard, 79350 Sexau (DE); Jaksic, Davorin, 79211 Denzlingen (DE); Kietz, Daniel, 79359 Riegel (DE); Märkle, Christoph, 79102 Freiburg (DE); Burger, Michael, 79263 Simonswald (DE); Vogelbacher, Armin, 79232 March (DE); Stingl, Michael, 79194 Heuweiler (DE); Schwarz, Simone, 79215 Elzach (DE)

(56) Entgegenhaltungen:
- DE-A1-102013 212 710
- US-A1- 2011 093 250
- US-A1- 2013 090 905
- J SHIEH ET AL: "The selection of sensors", PROGRESS IN MATERIALS SCIENCE., Bd. 46, Nr. 3-4, 1. Januar 2001 (2001-01-01), Seiten 461-504, XP055303727, GB ISSN: 0079-6425, DOI: 10.1016/S0079-6425(00)00011-6
- R. Agner: "Choose the Right Object Detection Sensor", SICK AG , 31. Mai 2015 (2015-05-31), Seiten 1-34, XP055303820, Gefunden im Internet: URL:http://sickusablog.com/wp-content/uplo ads/2014/09/2013_12-Choose-the-Right-Senso r.pdf [gefunden am 2016-09-19]

## Beschreibung

Die Erfindung betrifft ein System zum Simulieren von Sensoren, die insbesondere eine Entfernung zwischen Sensor und einem Objekt messen, geometrische Abmessungen des Objekts messen, Positionen des Objekts messen, Material, Kontrast, Farbe, Lumineszenz, Glanz, Transparenz des Objekts, Polarisation des vom Objekt reflektierten Lichts messen oder Magnetfeldstärke messen.

Sensorhersteller haben eine Vielzahl von Sensoren, wie z.B. Lichtschranken, Reflexlichtschranken, Mehrstrahllichtschranken, Lichttaster, Lichtgitter, optische Zeilensensoren, Kamerasysteme, induktive oder kapazitive Näherungsschalter, magnetische Zylindersensoren, magnetische Näherungssensoren, Radarabstandssensoren und womöglich auch weitere andere Messeinrichtungen im Angebot. Jeder einzelne Typ wird dabei noch in vielfachen Varianten angeboten. Zum Beispiel kann eine einfache Lichtschranke in Varianten angeboten werden, die verschiedene Entfernungen überbrücken, die mit unterschiedlichen Wellenlängen-längen arbeiten, die mit unterschiedlichen Leistungen arbeiten und so weiter und so fort. Sensorhersteller haben deshalb viele hundert oder viele tausende verschiedene Sensoren und Messgeräte im Programm. Alle diese Sensoren und Messgeräte werden nachfolgend einfach als "Sensoren" bezeichnet.

Für einen Kunden, der zum Beispiel eine neue Maschine aufbauen will und dafür einzelne spezielle dieser vielen Sensoren benötigt, wobei der Kunde selbst die Sensoren, deren Leistungsfähigkeit und die Breite der möglichen Applikationen gar nicht im Detail kennt, besteht deshalb das Problem, die richtigen, geeigneten Sensoren für seine Applikation zu finden.

Da heutzutage große Datenbanken mit den heutigen Computern und mobil über das Internet abrufbar sind, sind sogenannte Produktfinder bekannt, mit denen hinterlegte Sensoreigenschaften in einem Programm zum Beispiel durch ein Drop-Down-Menu stufenweise ausgewählt werden können, so dass am Ende der möglichen Auswahlen einzelne oder mehrere Sensoren aufgelistet werden.

Das ist aber nichts anderes, als eine Auswahl über die Datenblätter der Sensoren. Ob die so ausgewählten Sensoren ihre Funktion in der geplanten Applikation auch erfüllen, hängt wieder davon ab, welche Sachkenntnisse die auswählende Person bezüglich der Applikation und bezüglich der Sensoren hat und davon, wie intelligent sie diese Sachkenntnisse verbinden kann.

Aus der DE 10 2013 212 710 A1 und der US 2013/0090905 A1 sind Systeme zum Simulieren von Sensoren bekannt.

Ausgehend von diesem Stand der Technik ist es Aufgabe der Erfindung, ein System bereitzustellen, mit dem die Sensoren selbst, also deren Funktion in einer Anwendung, simuliert werden können, mit dem Ziel zu prüfen, welcher Sensor die Applikation erfüllt bzw. am besten erfüllt.

Diese Aufgabe wird gelöst durch ein System mit den Merkmalen des Anspruchs 1, ein Verfahren nach Anspruch 5 und ein entsprechendes Computerprogramm und Computerprogrammprodukt.

Das erfindungsgemäße System zum Simulieren von Sensoren umfasst:
- eine Speichereinheit, in der Sensormodelle mit Sensormodell-eigenschaften gespeichert sind,
- eine Auswerteeinheit zum Simulieren aller Sensoren
- und das Simulieren erfolgt anhand der gespeicherten Sensormodelle,
wobei
- die Sensoren ausgebildet sind geometrische Abmessungen des Objekts zu messen, Positionen des Objekts zu messen, Material, Kontrast, Farbe, Lumineszenz, Glanz, Transparenz des Objekts, Polarisation des vom Objekt reflektierten Lichts zu messen oder Magnetfeldstärke zu messen,
- eine Eingabeeinheit vorgesehen ist zum Eingeben einer beschränkten Anzahl von physikalischen Parametern, die eine oder mehrere vordefinierte Sensoranforderungen parametrieren, wobei zur Unterstützung beim Eingeben der Parameter auf einer Anzeigeeinheit eine stilisierte, typische Beispielanwendungssituation mittels grafischer Symbole dargestellt ist,
- die Auswerteeinheit ausgebildet ist, die Simulation dahingehend durchzuführen, ob die simulierten Sensoren die parametrierten Sensoranforderungen erfüllen,
- jeder Sensormodelleigenschaft wenigstens eine Abbildungsvorschrift zugeordnet ist, mit der eine oder mehrere der Sensormodelleigenschaften auf eine der parametrierten Sensoranforderungen abgebildet wird
- und die Auswerteeinheit eine Bewertungseinheit umfasst, die bewertet, ob ein Sensormodell die parametrierten Sensoranforderungen erfüllt
- wobei eine Anzeigeeinheit vorgesehen ist, zum Anzeigen wenigstens eines Teils des Bewertungsergebnisses und die Anzeigeeinheit eine Rangliste der simulierten Sensoren entsprechend dem Bewertungsergebnis ausgibt.

Der Sensor wird also in einem beschriebenen Applikationsumfeld durch ein geeignetes Simulationsmodell simuliert. Der besondere Vorteil der Erfindung ist, dass durch die Simulation eine anwendungsoptimale Zuordnung von Sensor-modellen zu den vorgegebenen Anforderungen an eine Applikation erfolgt, wobei die Sensormodelle alle in der Speichereinheit Vorhandenen umfassen. Damit werden nicht nur alle Sensoren berücksichtigt, so dass stets die am besten passenden Sensoren oder der passendste Sensor durch die Simulation gefunden werden kann, sondern es sind für die Anwendung der Simulation auch keine umfangreichen Spezialkenntnisse über die Sensoren notwendig. Durch die hinterlegten Sensormodelle braucht der Anwender, der die Sensoren in seiner Anwendung einsetzen will, keine besondere Sachkenntnis über die Sensoren. Der "best match" zwischen Applikation und Sensor wird durch die Simulation automatisch gefunden. Dazu dienen in erster Linie die sensormodellspezifischen Abbildungsvorschriften. In diesen Abbildungsvorschriften steckt das Expertenwissen über die Sensormodelle und die Anwendungsfelder. Der große Vorteil ist, dass dieses Expertenwissen nur einmal zum Zeitpunkt des Aufstellens der Abbildungs-vorschriften angewandt werden muss und nachfolgend bei jeder Simulation immer wieder ohne Aufwand zur Verfügung steht. Damit wird erheblicher Aufwand eingespart. Auch wird das Expertenwissen, was in den genannten Abbildungs-vorschriften steckt, mit dem erfindungsgemäßen System quasi konserviert und kann im Laufe der Zeit durch Hinzufügen weiterer Sensormodelle mit weiteren Sensormodelleigenschaften ergänzt werden. Derjenige, der die Simulation durchführt, benötigt dieses Expertenwissen nicht, sondern nutzt es, ohne selbst die Kenntnisse besitzen zu müssen.

Projekte, wie die Ausrüstung von großen Maschinen mit einer Vielzahl verschiedener Sensoren, können mit dem erfindungsgemäßen System erheblich schneller abgewickelt werden und das von Personen, die nicht selbst über Expertenwissen verfügen. Dieser Zeitgewinn wird größer, je größer die Gesamtapplikation, z.B. ganze Fertigungsstraßen, ist.

Erfindungsgemäß kann durch die Simulation nicht nur festgestellt werden, ob ein Sensor die parametrierten Sensoranforderungen erfüllt, sondern durch die Bewertungseinheit kann auch bewertet werden, wie gut ein Sensormodell die parametrierten Sensoranforderungen erfüllt. Es kann somit eine Rangliste der simulierten Sensoren entsprechend dem Bewertungsergebnis ausgegeben werden, damit der Anwender die am besten zur Applikation passenden Sensoren erfährt.

Zur Ausgabe des Simulationsergebnisses ist eine Anzeigeeinheit vorgesehen, zum Anzeigen wenigstens eines Teils des Bewertungsergebnisses. So kann z.B. die Rangliste angezeigt werden.

In Weiterbildung der Erfindung kann als Abbildungsvorschrift eine analytische Funktion und/oder ein in der Datenbank hinterlegtes Diagramm und/oder eine Look-up Tabelle hinterlegt sein. Darin steckt dann das echte Expertenwissen. Ein Beispiel soll dies verdeutlichen. Beispielsweise kann in der Applikation gefordert sein, dass ein Strichcode einer minimalen Strichstärke d und Strichabstand d1 bei einem Kontrast K und bei vorgegebener Geschwindigkeit v innerhalb eines Abstandsbereiches detektiert werden soll. Für einen punktförmig abtastenden Sensor könnte eine hinterlegte Abbildungsvorschrift aus der Verknüpfung verschiedener funktionaler Zusammenhänge wie Lichtfleckgröße über Tastabstand; Lichtfleckgröße im Verhältnis zur Strichbreite und Lücke und dem Verhältnis von Kontrastauflösung des Sensors zum vorherrschenden Kontrast der Applikation (Strichcode) bestehen. Der Vorteil der Erfindung liegt dann gerade darin, dass solche komplexen, funktionalen Zusammenhänge durch die hinterlegten Abbildungsvorschriften berücksichtigt werden können.

Die Abbildungsvorschriften können auch den einfachsten Fall umfassen, wenn nämlich eine einzelne Sensormodelleigenschaft mit einer Sensoranforderung identisch ist. Das ist zwar nicht das Thema der Erfindung, aber es soll dennoch hier erwähnt werden, um zu zeigen, dass die Erfindung auch das Bekannte abdecken kann. In diesem Fall ist die Abbildungsvorschrift eine 1-zu-1 Zuordnung. Dies kann z.B. dann der Fall sein, wenn es sich bei der Sensormodelleigenschaft um einen Entfernungsbereich handelt, innerhalb der der Sensor arbeiten kann und bei der zugeordneten Sensoranforderung um die zu messende Entfernung zwischen Sensor und Objekt. Dann steckt hinter der Sensormodelleigenschaft und der parametrierten Sensoranforderung derselbe physikalische Parameter, nämlich die Entfernung.

In Weiterbildung der Erfindung kann die Simulationsvielfalt, die sich aus der Anzahl hinterlegter Sensormodelle und den zugehörigen Sensormodelleigenschaften ergibt, von Vorneherein reduziert werden. Indem nämlich ein Vorfilter vorgesehen ist, der vor Beginn der Simulation anwählbar ist und über den eine Vorauswahl der Sensormodelle erfolgt. Ein Beispiel wäre z.B. die Auswahl des physikalischen Prinzips, also ob der Sensor magnetisch, optisch oder kapazitiv arbeitet. So ist es denkbar, dass ein Anwender des Simulationssystems Vorkenntnisse hat und schon zuvor weiß, welche grundsätzliche Art Sensormodell, wie optischer Sensor, induktiver Sensor, kapazitiver Sensor, in Frage kommt. Eine solche Vorfilterung reduziert die Simulationsvielfalt erheblich und führt deshalb schneller zum Ergebnis.

Andere Vorfilter, die auch eine Reduzierung der Simulationsvielfalt bezwecken, sind denkbar. So kann mit dem Vorfilter eine Auswahl zum Beispiel bzgl. Temperatur, Schmutz, Feuchte abgefragt werden, die dann ebenfalls eine Eingrenzung erlauben.

In gleicher Weise kann nach der Simulation, also nach Erhalt des Simulationsergebnisses, ein Nachfilter anwählbar sein, mit dem weitere Parameter festlegbar sind, wie z.B. Steckerart, pnp- oder npn-Ausgang, Leitungslänge, etc., die nicht einer Simulation bedürfen.

Die Erfindung betrifft somit auch ein Simulationsverfahren zum Simulieren von Sensoren, die insbesondere eine Entfernung zwischen Sensor und einem Objekt messen, geometrische Abmessungen des Objekts messen, Positionen des Objekts messen, Material, Kontrast, Farbe, Lumineszenz, Glanz, Transparenz des Objekts, Polarisation des vom Objekt reflektierten Lichts messen oder Magnetfeldstärke messen. Das Simulationsverfahren, das auf dem vorbeschriebenen System ausgeführt wird, umfasst die Schritte:
- Eingeben einer beschränkten Anzahl von physikalischen Parametern, die eine oder mehrere vordefinierte Sensoranforderung parametrieren, in eine Eingabeeinheit des Systems,
- Simulieren der Sensoren mittels Sensormodellen, die in einer Speichereinheit gespeichert sind und Sensormodelleigenschaften aufweisen,
- wobei das Simulieren in einer Auswerteeinheit anhand der gespeicherten Sensormodelle erfolgt, wobei jeder Sensormodelleigenschaft wenigstens eine Abbildungsvorschrift zugeordnet ist, mit der eine oder mehrere der Sensormodelleigenschaften auf eine der parametrierten Sensoranforderungen abgebildet wird,
- Bewerten in einer Bewertungseinheit des Systems, ob ein Sensormodell die parametrierten Sensoranforderungen erfüllt.

In einem weiteren Schritt wird zusätzlich bewertet, wie gut ein Sensormodell die parametrierten Sensoranforderungen erfüllt. Damit kann eine Ergebnisrangliste ausgegeben werden, welches Sensormodell und damit Sensor die Anforderungen am Besten erfüllt.

Im Folgenden wird die Erfindung anhand eines Ausführungsbeispiels unter Bezugnahme auf die Zeichnung im Einzelnen erläutert. In der Zeichnung zeigen:
- Fig. 1: eine schematische Darstellung eines erfindungsgemäßen Systems;
- Fig. 2: eine schematische Darstellung einer stilisierten Grafik zur Eingabe von Parametern vordefinierter Sensoranforderungen;
- Fig. 3: eine schematische Darstellung zur Verdeutlichung der Abbildungsvorschriften zur Zuordnung der Sensormodelleigenschaften zu den parametrierten Sensoranforderungen.

Mit dem erfindungsgemäßen System zum Simulieren von Sensoren sollen letztendlich für eine aktuelle Applikation geeignete Sensoren aus einer Vielzahl von Sensoren bestimmt werden. Mit der Simulation werden also Sensormodelle simuliert unter Berücksichtigung applikationsspezifischer Sensoranforderungen, die sich aus einer aktuellen Applikation ergeben. Die applikationsspezifischen Sensoranforderungen sind durch physikalische Parameter bestimmt. Im einfachsten Fall kann eine applikationsspezifische Sensoranforderung zum Beispiel sein, dass der Sensor im Abstandsbereich von 1 m bis 2 m messen können muss. Dann ist der Parameter, der diese Sensoranforderung parametrisiert, ein Entfernungswert.

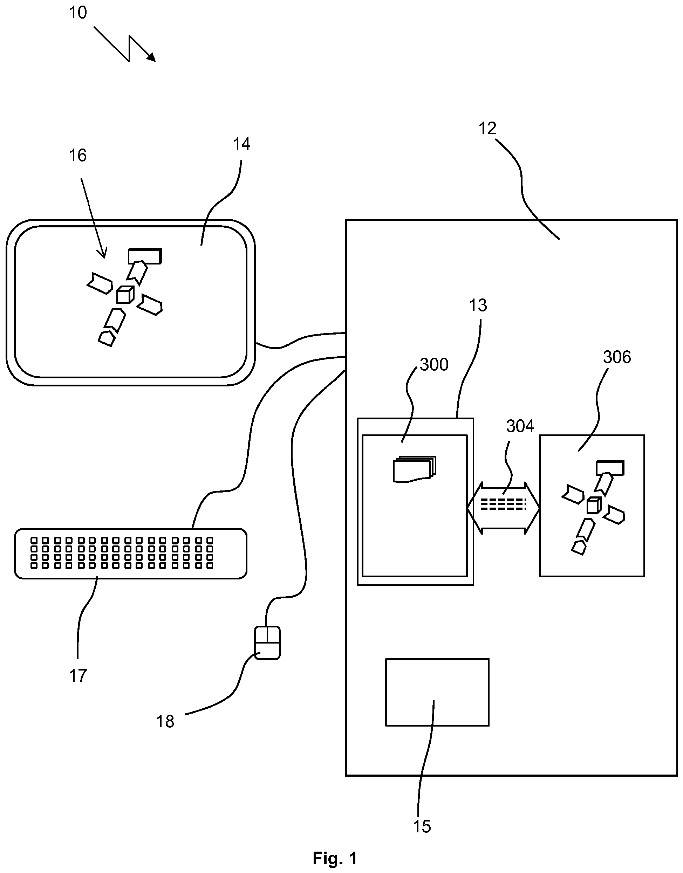

Das System 10 kann durch einen Computer 10 gebildet sein, der auf verschiedenste, bekannte Arten ausgestaltet sein kann, beispielsweise als Desktop-PC wie in Fig. 1 beispielhaft dargestellt, als Tablet-PC, Smartphone oder dergleichen mehr. Das System 10 weist zumindest eine Eingabeeinheit 17, 18, eine Speichereinheit 13 und eine Auswerteeinheit 12 sowie eine Anzeigeeinheit 14 auf. Andere Zusammensetzungen des Systems 10 sind möglich. Z.B. könnte die Speichereinheit auf einem externen Server ("Cloud") eingerichtet sein.

Zur Unterstützung beim Eingeben der Parameter wird auf der Anzeigeeinheit 14 eine stilisierte, typische Beispielanwendungssituation 16 mittels grafischer Symbole, die nachfolgend noch erläutert werden, dargestellt. Diese Beispielanwendungs-situation 16 steht stellvertretend für eine Vielzahl von Applikationen, weshalb sie stilisiert gehalten ist. Die einzelnen Symbole repräsentieren physikalische Objekte und/oder Eigenschaften der Applikation. Ziel der Darstellung ist die optimale graphische Unterstützung bei der Erfassung der Parameter für eine Sensor-anforderung.

Die Vielzahl der Parameter dürfen den Anwender nicht überfordern und verlangen eine optimal angepasste graphische Unterstützung, um z.B. Objektdimensionen korrekt zur Lage des Sensors und der Bewegungsrichtung des Objekts zu erfassen. Die Anwendungsszene ist dann geeignet dargestellt, wenn sie den Sachverhalt möglichst korrekt wiedergibt und eine hohe Identifikation beim Anwender hervorruft.

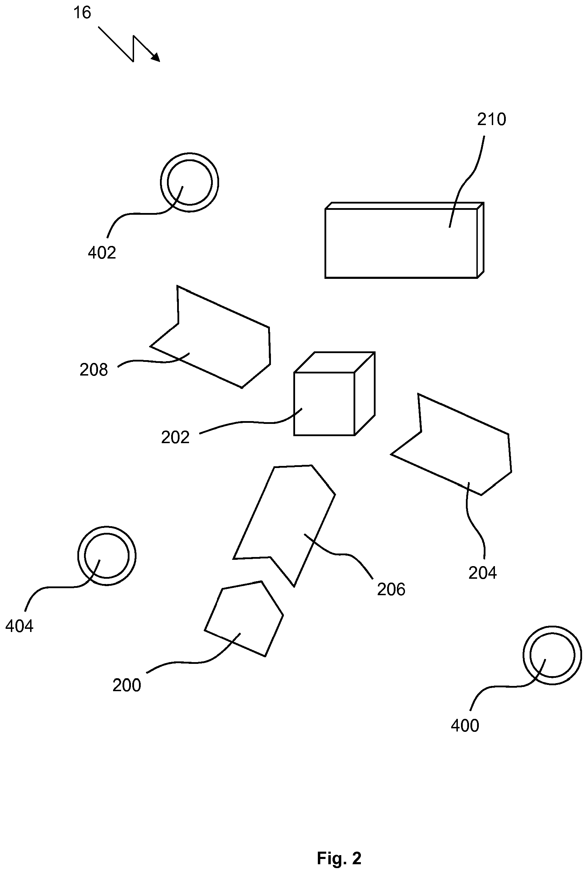

Die Beispielanwendungssituation 16 ist in Fig. 2 vergrößert dargestellt und wird anhand der Fig. 2 näher erläutert. Ein Symbol 200 steht stellvertretend für den (oder die) Sensor(en), der die aktuelle Applikation bedienen soll. Die Applikation selbst wird dargestellt durch Symbole 202, 204 und 206. Das Symbol 202 steht stellvertretend für ein zu detektierendes Objekt, das Symbol 204 für eine Bewegungsrichtung des Objekts und das Symbol 206 für einen Abstand zwischen Sensor 200 und dem Objekt 202. Über das Symbol 206 können der Abstand selbst, aber auch Vordergrundinformationen, wie z.B. Einbaubedingungen, wie weiter unten erläutert, eingegeben werden.

Es können ergänzend weitere Symbole vorgesehen sein, wie z.B. ein Symbol 208 für eine Leistungsanforderung, worunter Objektgeschwindigkeit, Auflösung und Genauigkeit verstanden wird und ein Symbol 210 für die Beschreibung des Raumes hinter dem Objekt (Applikationshintergrund).

Zu jedem Symbol der Applikation sind Parametersätze definiert, wobei die Parameter wie oben erwähnt Sensoranforderungen, also physikalischen Eigenschaften der aktuellen Applikation, für die ein Sensor simuliert werden soll, entsprechen und somit die Applikation spezifizieren. Die Parametersätze können durch geeignete Defaultwerte vorbelegt werden.

Zum Objektsymbol 202 ist ein Objektparametersatz definiert, der einen oder mehrere der folgenden Parameter umfassen kann: "minimale Objektlänge in Bewegungsrichtung", "maximale Objektlänge in Bewegungsrichtung" , "minimale Objektbreite", "maximale Objektbreite", "minimale Objekthöhe", "maximale Objekthöhe", "Positionstoleranz", "Material", "Kontrast", "Farbe", "Lumineszenz", "Glanz", "Transparenz", "Depolarisationsfähigkeit", "Fokussierfähigkeit", "Magnetfeldstärke".

Um die Objektdimensionen in Bezug auf den Sensor und die Bewegungsrichtung korrekt erfassen zu können, sind nachgelagerte grafische Darstellungen sinnvoll, mit denen die Länge, Breite und Höhe von Objekten eindeutig, auch bei zweidimensionalen Sensoreinrichtungen ermöglicht wird. Das ist grob durch die dreidimensionale Darstellung des Objektsymbols 202 als Box angedeutet.

Zum Bewegungsrichtungssymbol 204 ist ein Richtungsparametersatz definiert und zum Objektabstandssymbol 206 ein Abstandsparametersatz. Der Abstandsparametersatz kann einen oder mehrere der folgenden Parameter umfassen: "Abstand Sensor-Objekt", "Abstand Sensor-Reflektor" und "Objektführungstoleranzen". Auch können über das Objektabstandsymbol 206 die Eigenschaften des Raumes vor dem Objekt (Vordergrundinformationen) und/oder Einbau-bedingungen eingegeben werden. Die Einbaubedingen sind insbesondere für induktive Sensoren relevant, denn bei diesen Sensoren ist es entscheidend, in welchem Abstand und in welcher Größe sich insbesondere metallene Gegenstände im Bereich des Frontend des Sensors befinden.

Der vorgenannte Hintergrundparametersatz kann einen Parameter "Hintergrundart" umfassen. Damit können unterschiedliche Hintergrundarten verschiedener Sensorsysteme beschrieben werden. So ist in einem getrennte Sender-/Empfänger-System der Hintergrund durch den Empfänger gebildet. Bei Gabelsensoren ist der Hintergrund durch einen der Gabelzweige gebildet und bei Reflexsystemen durch einen Reflektor. Ein anderer Parameter kann "Fremdlicht" sein. Dadurch können auch Hintergrundeigenschaften, wie z.B. störendes Fremdlicht oder undefinierte Reflexionen an Grenzflächen, beschrieben werden, woraus sich beispielsweise die Auswahl spezifischer Lichttaster (mit oder ohne Hintergrundausblendung) ableitet.

Mit diesem Satz an Symbolen und zugehörigen Parametern lässt sich der größte Teil aller möglichen Applikationen erfassen. Die Parameter werden in einem Parameterspeicher 306 gespeichert. Die Eingabe kann durch verschiedenste bekannte Arten erfolgen, beispielsweise durch Klicken auf ein Symbol, wodurch sich ein drop-down Auswahlmenü öffnet, oder die Eingabe von konkreten Zahlen, z.B. Eingabe Sensor-Objekt-Abstand, beispielsweise durch direkte Zahleneingabe in ein entsprechendes Fenster, dass sich durch Klick auf das entsprechende Symbol 206 öffnet oder durch Ziehen grafischer Symbole.

In der Speichereinheit 13 sind Sensormodelle mit Sensormodelleigenschaften gespeichert. Die Erfindung betrifft die Situation, wenn die parametrierten Sensoranforderungen nicht identisch sind mit Sensormodelleigenschaften, denn genau für diese Situation wird ein Mechanismus benötigt, um Sensoranforderungen und Sensormodelleigenschaften in Einklang zu bringen. Das schließt nicht aus, dass es dennoch parametrierte Sensoranforderungen geben kann, die 1-zu-1 einer Sensormodelleigenschaft entsprechen. Dies trifft zum Beispiel auf den Abstand Sensor-Objekt als Sensoranforderung zu, der einer Sensormodelleigenschaft "Messbereich" entspricht. Das ist aber ein trivialer Fall, für den alleine keine Simulation benötigt würde.

Die Erfindung beschäftigt sich also mit den Situationen, in denen diese trivialen Fälle nicht oder nicht alleine vorliegen. Deshalb ist die Auswerteeinheit 12 vorgesehen, mit der alle gespeicherten Sensoren simuliert werden können. Es wird also in der Simulation abgeprüft, ob die zur Verfügung stehenden Sensoren unter Anwendung des zugehörigen Sensormodells die parametrierten Sensor-anforderungen erfüllen. Da wie bereits erwähnt die parametrierten Sensor-anforderungen nicht identisch sind mit den Sensormodelleigenschaften, erfolgt erfindungsgemäß das Simulieren in der Art, dass jeder Sensormodelleigenschaft wenigstens eine Abbildungsvorschrift zugeordnet ist, mit der eine oder mehrere der Sensormodelleigenschaften auf eine der parametrierten Sensoranforderungen abgebildet wird.

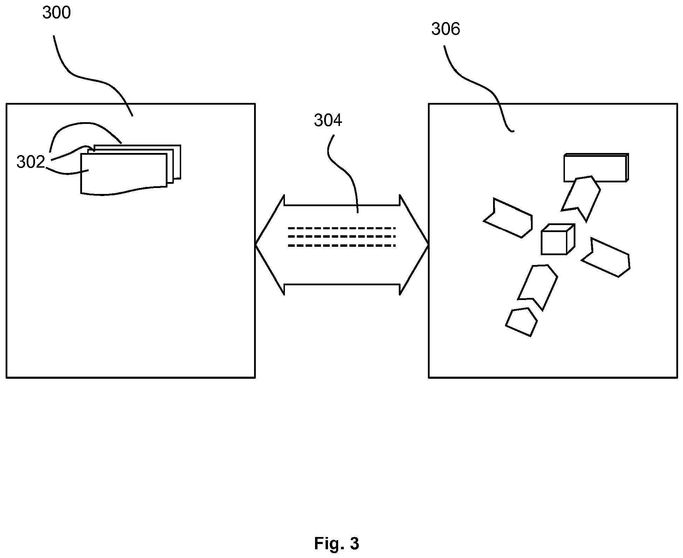

Das ist in Fig. 3 plakativ dargestellt. Dabei werden die Sensormodelleigenschaften 302, die in einer Datenbank 300 gespeichert sind, mittels der dem jeweiligen Sensormodell zugeordneten Abbildungsvorschrift 304 auf die im Parameterspeicher 306 gespeicherten Parameter der Applikation abgebildet. Die sensormodellspezifischen Abbildungsvorschriften 304 wurden zuvor aufgestellt. Das bedeutet, dass auf Basis der hinterlegten Abbildungsvorschriften 304 Zuordnungen zwischen den gespeicherten Parametern und den Sensormodelleigenschaften 302 bestehen. Nach Auswahl oder Eingabe der Parameter durch Eingabe am Computer 10 werden durch den Computer 10 jetzt die Sensormodelle durchsimuliert.

Die Abbildungsvorschriften 304 wurden in Kenntnis aller auswählbaren Parameter aufgestellt und beinhalten somit das Expertenwissen über die Sensoren und Anwendungsfelder. Eine Abbildungsvorschrift 302 kann als eine analytische Funktion und/oder ein in der Datenbank hinterlegtes Diagramm und/oder eine Look-up Tabelle hinterlegt sein. Beispielsweise kann in der Applikation gefordert sein, dass ein Strichcode einer minimalen Strichstärke d und Strichabstand d1, bei einem Kontrast K und bei vorgegebener Geschwindigkeit v innerhalb eines Abstands-bereiches detektiert werden soll. Für einen punktförmig abtastenden Sensor könnte eine hinterlegte Abbildungsvorschrift aus der Verknüpfung verschiedener funktionaler Zusammenhänge wie Lichtfleckgröße über Tastabstand, Lichtfleck-größe im Verhältnis zur Strichbreite und Lücke und dem Verhältnis von Kontrast-auflösung des Sensors zum vorherrschenden Kontrast der Applikation (Strichcode) bestehen. Der Vorteil solcher Abbildungsvorschriften liegt gerade darin, dass komplexe, funktionale Zusammenhänge in der Simulation berücksichtigt werden können.

In diesen aufwändigeren Abbildungsvorschriften, die jede für sich zuvor einmal aufgestellt werden müssen, ist das Expertenwissen über die Sensoren und deren Verwendbarkeit enthalten.

Mit der Simulation werden also alle Sensormodelle durchgegangen. Erfindungsgemäß ist schließlich eine Bewertungseinheit 15 vorgesehen, mit der bewertet wird, ob ein Sensormodell die parametrierten Sensoranforderungen erfüllt. In Weiterbildung kann die Bewertungseinheit 15 auch bewerten "wie gut" ein Sensormodell die Sensoranforderungen erfüllt. Das Ergebnis ist dann eine "Rangliste" von Sensormodellen, die auf der Anzeigeeinheit 14 dargestellt werden kann. Es sind dann Sensormodelle angezeigt, die die parametrierten Sensoranforderungen und damit die Applikation bestmöglich erfüllen.

Neben den gefundenen Sensoren kann auch notwendiges Zubehör angezeigt werden, so dass der Nutzer erkennen kann, dass zu dem gefundenen Sensor noch das angezeigte Zubehör benötigt wird, um die Applikation zu erfüllen. Die Funktionalität und Leistungsfähigkeit von Sensoren ist nämlich oftmals direkt mit den spezifischen Eigenschaften zugeordneter Komponenten "Zubehör" verknüpft. Sensor und "Zubehör" bilden dann ein Gesamtsystem. Auf der Basis der Parameter werden in solchen Fällen also Systeme gefunden, die aus dem eigentlichen Sensor und weiteren Komponenten bestehen. Beispielsweise werden die Systemeigenschaften durch "Zubehör" wie Reflektoren (Größe und Technologie) sowie unterschiedlich lange Lichtleiter (Dämpfung) oder spezifische Magnete (Magnetfeldstärke) beeinflusst. Neben den gefundenen Sensoren soll in der Ergebnisdarstellung deshalb auch zugeordnetes Zubehör angezeigt werden. Beispielsweise könnte eine bestimmte Reflexionslichtschranke als Ergebnis vorliegen, die mit einem Tripelreflektor als Reflektor eine andere, nämlich viel größere Reichweite hat, als mit einer Reflexionsfolie als Reflektor. Die zusätzliche Angabe des benötigten Reflektortyps ist in einem solchen Fall deshalb sinnvoll und hilfreich.

Weiter kann ein sogenannter Vorfilter vorgesehen sein, der über ein Symbol 400 oder 402 aktiviert werden kann. Die Symbole 400 und 402 stehen für zwei verschiedenartige Vorfilter.

Der eine Vorfilter 400 dient dazu, durch wenige spezifische Merkmalsabfragen (Parameter) die möglichen Technologien einzuschränken und aus dem a-priori Wissen über die spezifische Technologie, die Applikationserfassung durch spezifischere Fragenstellungen zu vereinfachen. Wenn der Anwender sich auf solche spezifischen Parameter beschränkt, kann wenn diese Parameter in einer vordefinierten Zuordnung zu den Sensormodellen stehen, das System nur diese zugeordneten Sensormodelle für die Simulation nutzen und andere Sensormodelle verwerfen. Dadurch ist der Simulationsumfang reduziert und die Simulation läuft erheblich schneller ab. Es ist deshalb sinnvoll, wenn die Symbole der Beispielanwendungssituation 16 erst dann aktivierbar sind, wenn ein solcher Vorfilter durchlaufen wurde.

Weiter kann ein anderer Vorfilter 402 vorgesehen sein, der einer Experten- oder Fortgeschrittenenfunktion entspricht. Dieser Vorfilter setzt Kenntnisse der Sensormodelle voraus, denn er erlaubt eine Reduktion der Auswahl von möglichen Sensormodellen. Der Expertenanwender kann damit Sensormodelle, die die Simulation nutzen soll, direkt anwählen. Das reduziert wiederum den Simulationsaufwand und Rechenzeit und aufgrund der vorgenannten Zuordnungen der Sensoranforderungen zu den Sensormodellen kann das auch die Menge der einzugebenden Parameter reduzieren. Dieser Vorfilter ist eine Art Short-cut zur gewünschten Technologie für den "advanced user". Mit ihm kann also eins oder mehrere der Sensormodelle, wie z.B. optische Sensoren oder induktive Sensoren, direkt ausgewählt werden. Alle anderen Sensormodelle werden in der Simulation dann nicht weiter betrachtet.

Nach der Simulation kann ein sogenannter Nachfilter über ein Symbol 404 anwählbar sein. Mit diesem Nachfilter, sind weitere Parameter festlegbar, wie z.B. Steckerart, pnp- oder npn-Ausgang, Leitungslänge und dergleichen. Auch kann noch ein weiteres Nachfilter vorgesehen sein bzgl. Merkmalen, die nicht mehr die aktuelle Applikation betreffen, sondern nicht-sensorspezifische Kundenwünsche, wie Produktneuheit, Preis, Verfügbarkeit, regionale Besonderheiten und dergleichen mehr.

## Patentansprüche

1. System zum Simulieren von Sensoren,
- mit einer Speichereinheit (13), in der Sensormodelle mit Sensormodelleigenschaften (302) gespeichert sind,
- mit einer Auswerteeinheit (12) zum Simulieren aller Sensoren
- und das Simulieren anhand der gespeicherten Sensormodelle erfolgt,
- und die Sensoren ausgebildet sind, geometrische Abmessungen des Objekts zu messen, Positionen des Objekts zu messen, Material, Kontrast, Farbe, Lumineszenz, Glanz, Transparenz des Objekts, Polarisation des vom Objekt reflektierten Lichts zu messen oder Magnetfeldstärke zu messen,
- eine Eingabeeinheit (17, 18) vorgesehen ist zum Eingeben einer beschränkten Anzahl von physikalischen Parametern, die eine oder mehrere vordefinierte Sensoranforderungen parametrieren, wobei zur Unterstützung beim Eingeben der Parameter auf einer Anzeigeeinheit (14) eine stilisierte, typische Beispielanwendungssituation (16) mittels grafischer Symbole dargestellt ist,
- wobei die Auswerteeinheit (12) ausgebildet ist, die Simulation dahingehend durchzuführen, ob die simulierten Sensoren die parametrierten Sensoranforderungen erfüllen,
- wobei jeder Sensormodelleigenschaft (302) wenigstens eine Abbildungsvorschrift (304) zugeordnet ist, mit der eine oder mehrere der Sensormodelleigenschaften (302) auf eine der parametrierten Sensoranforderungen abgebildet wird
- und die Auswerteeinheit (12) eine Bewertungseinheit (15) umfasst, die bewertet, ob ein Sensormodell die parametrierten Sensoranforderungen erfüllt,
- wobei eine Anzeigeeinheit vorgesehen ist, zum Anzeigen wenigstens eines Teils des Bewertungsergebnisses und die Anzeigeeinheit eine Rangliste der simulierten Sensoren entsprechend dem Bewertungsergebnis ausgibt.

2. System nach Anspruch 1, **dadurch gekennzeichnet, dass** die Bewertungseinheit bewertet, wie gut ein Sensormodell die parametrierten Sensoranforderungen erfüllt.

3. System nach einem der vorhergehenden Ansprüche, wobei die Abbildungsvorschrift eine analytische Funktion und/oder ein in der Speichereinheit hinterlegtes Diagramm und/oder eine Look-up Tabelle ist.

4. System nach einem der vorhergehenden Ansprüche, wobei zu bestimmten Parametern nur vordefinierte Werte eingegeben werden können.

5. Verfahren zum Simulieren von Sensoren mit den Schritten,
- Simulieren aller Sensoren in einer Auswerteeinheit anhand von Sensormodellen, die mit Sensormodelleigenschaften (302) in einer Speichereinheit (13) gespeichert sind und die Sensoren geometrische Abmessungen eines Objekts, Positionen des Objekts, Material, Kontrast, Farbe, Lumineszenz, Glanz, Transparenz des Objekts, Polarisation des vom Objekt reflektierten Lichts oder Magnetfeldstärke messen können,
- Eingeben einer beschränkten Anzahl von physikalischen Parametern, die eine oder mehrere vordefinierte Sensoranforderungen parametrieren, in eine Eingabeeinheit (17, 18), wobei zur Unterstützung beim Eingeben der Parameter auf einer Anzeigeeinheit (14) eine stilisierte, typische Beispielanwendungssituation (16) mittels grafischer Symbole dargestellt wird,
- wobei die Simulation in der Auswerteeinheit (12) dahingehend durchgeführt wird, ob die simulierten Sensoren die parametrierten Sensoranforderungen erfüllen,
- wobei jeder Sensormodelleigenschaft (302) wenigstens eine Abbildungsvorschrift (304) zugeordnet ist, mit der eine oder mehrere der Sensormodelleigenschaften (302) auf eine der parametrierten Sensoranforderungen abgebildet wird,
- wobei in einer Bewertungseinheit (15) bewertet wird, ob ein Sensormodell die parametrierten Sensoranforderungen erfüllt,
- wobei auf einer Anzeigeeinheit wenigstens ein Teil des Bewertungsergebnisses angezeigt wird und die Anzeigeeinheit eine Rangliste der simulierten Sensoren entsprechend dem Bewertungsergebnis ausgibt.

6. Computerprogramm mit Anweisungen, die bei Ausführung durch einen Computer den Computer veranlassen, ein Verfahren nach Anspruch 5 auszuführen.

7. Computerprogrammprodukt mit Programmcode-Mitteln, die auf einem computerlesbaren Datenträger gespeichert sind und derart eingerichtet sind, dass der Computer nach Laden des Computerprogramms in den Computer alle Verfahrensschritte des Anspruchs 5 ausführt, wenn das Computerprogramm auf dem Computer ausgeführt wird.

## Claims

1. System for simulating sensors,
- with a memory unit (13) in which sensor models with sensor model properties (302) are stored,
- with an evaluation unit (12) for simulating all sensors,
- and the simulation is done using the stored sensor models,
- and the sensors are designed to measure geometric dimensions of the object, measure positions of the object, measure material, contrast, colour, luminescence, gloss, transparency of the object, polarisation of the light reflected from the object or measure magnetic field strength,
- an input unit (17, 18) is provided for inputting a limited number of physical parameters which parameterise one or more predefined sensor requirements, wherein a stylised, typical example application situation (16) is shown by means of graphic symbols on a display unit (14) to assist in inputting the parameters,
- the evaluation unit (12) being designed to carry out the simulation to determine whether the simulated sensors fulfil the parameterised sensor requirements,
- wherein each sensor model property (302) is associated with at least one mapping rule (304) by which one or more of the sensor model properties (302) is mapped to one of the parameterised sensor requirements,
- and the evaluation unit (12) comprises an evaluation unit (15) which evaluates whether a sensor model fulfils the parameterised sensor requirements,
- wherein a display unit is provided for displaying at least a part of the evaluation result and the display unit outputs a ranking list of the simulated sensors according to the evaluation result.

2. System according to claim 1, **characterised in that** the evaluation unit evaluates how well a sensor model fulfils the parameterised sensor requirements.

3. System according to any one of the preceding claims, wherein the mapping rule is an analytical function and/or a diagram stored in the memory unit and/or a look-up table.

4. System according to any of the preceding claims, wherein only predefined values can be entered for certain parameters.

5. Method for simulating sensors with the steps,
- simulating all sensors in an evaluation unit on the basis of sensor models which are stored with sensor model properties (302) in a memory unit (13) and the sensors can measure geometric dimensions of an object, positions of the object, material, contrast, colour, luminescence, gloss, transparency of the object, polarisation of the light reflected by the object or magnetic field strength,
- entering a limited number of physical parameters parameterising one or more predefined sensor requirements into an input unit (17, 18), wherein a stylised, typical example application situation (16) is shown by means of graphic symbols on a display unit (14) to assist in entering the parameters,
- whereby the simulation is carried out in the evaluation unit (12) to determine whether the simulated sensors fulfil the parameterised sensor requirements,
- wherein each sensor model property (302) is associated with at least one mapping rule (304) by which one or more of the sensor model properties (302) is mapped to one of the parameterised sensor requirements,
- wherein an evaluation unit (15) evaluates whether a sensor model fulfils the parameterised sensor requirements,
- wherein at least a part of the evaluation result is displayed on a display unit and the display unit outputs a ranking list of the simulated sensors according to the evaluation result.

6. A computer program comprising instructions which, when executed by a computer, cause the computer to perform a method according to claim 5.

7. A computer program product comprising program code means stored on a computer-readable medium and arranged such that, after loading the computer program into the computer, the computer performs all the method steps of claim 5 when the computer program is executed on the computer.

## Revendications

1. Système de simulation de capteurs,
- avec une unité de mémoire (13) dans laquelle sont stockés des modèles de capteur avec des propriétés de modèle de capteur (302),
- avec une unité d'évaluation (12) pour simuler tous les capteurs,
- et la simulation est effectuée à l'aide des modèles de capteurs stockés,
- et les capteurs sont conçus pour mesurer les dimensions géométriques de l'objet, mesurer les positions de l'objet, mesurer la matière, le contraste, la couleur, la luminescence, la brillance, la transparence de l'objet, la polarisation de la lumière réfléchie par l'objet ou mesurer l'intensité du champ magnétique,
- une unité d'entrée (17, 18) est prévue pour entrer un nombre limité de paramètres physiques qui paramètrent une ou plusieurs exigences prédéfinies du capteur, dans laquelle une situation d'application typique stylisée (16) est représentée au moyen de symboles graphiques sur une unité d'affichage (14) pour aider à entrer les paramètres,
- l'unité d'évaluation (12) étant conçue pour effectuer la simulation afin de déterminer si les capteurs simulés répondent aux exigences des capteurs paramétrés,
- dans lequel chaque propriété de modèle de capteur (302) est associée à au moins une règle demise en correspondance (304) par laquelle une ou plusieurs des propriétés de modèle de capteur (302) sont mises en correspondance avec l'une des exigences de capteur paramétrées,
- et l'unité d'évaluation (12) comprend une unité d'évaluation (15) qui évalue si un modèle de capteur remplit les exigences de capteur paramétrées,
- dans lequel une unité d'affichage est prévue pour afficher au moins une partie du résultat de l'évaluation et l'unité d'affichage émet une liste de classement des capteurs simulés en fonction du résultat de l'évaluation.

2. Système selon la revendication 1, **caractérisé en ce que** l'unité d'évaluation évalue dans quelle mesure un modèle de capteur remplit les exigences paramétrées du capteur.

3. Système selon l'une quelconque des revendications précédentes, dans lequel la règle de mise en correspondance est une fonction analytique et/ou un diagramme stocké dans l'unité de mémoire et/ou une table de consultation.

4. Système selon l'une quelconque des revendications précédentes, dans lequel seules des valeurs prédéfinies peuvent être saisies pour certains paramètres.

5. Méthode pour simuler des capteurs avec les étapes,
- simuler tous les capteurs dans une unité d'évaluation sur la base de modèles de capteurs qui sont stockés avec des propriétés de modèles de capteurs (302) dans une unité de mémoire (13) et les capteurs peuvent mesurer les dimensions géométriques d'un objet, les positions de l'objet, le matériau, le contraste, la couleur, la luminescence, la brillance, la transparence de l'objet, la polarisation de la lumière réfléchie par l'objet ou l'intensité du champ magnétique,
- entrée d'un nombre limité de paramètres physiques paramétrant une ou plusieurs exigences prédéfinies du capteur dans une unité d'entrée (17, 18), dans laquelle une situation d'application typique stylisée (16) est représentée au moyen de symboles graphiques sur une unité d'affichage (14) pour aider à l'entrée des paramètres,
- dans lequel la simulation est effectuée dans l'unité d'évaluation (12) pour déterminer si les capteurs simulés remplissent les exigences de capteur paramétrées,
- dans lequel chaque propriété de modèle de capteur (302) est associée à au moins une règle de mise en correspondance (304) par laquelle une ou plusieurs des propriétés de modèle de capteur (302) sont mises en correspondance avec l'une des exigences de capteur paramétrées,
- dans lequel une unité d'évaluation (15) évalue si un modèle de capteur remplit les exigences de capteur paramétrées,
- dans lequel au moins une partie du résultat de l'évaluation est affichée sur une unité d'affichage et l'unité d'affichage produit une liste de classement des capteurs simulés en fonction du résultat de l'évaluation.

6. Programme informatique comprenant des instructions qui, lorsqu'elles sont exécutées par un ordinateur, amènent l'ordinateur à exécuter un procédé selon la revendication 5.

7. Produit de programme informatique comprenant des moyens de code de programme stockés sur un support lisible par ordinateur et agencés de telle sorte que, après avoir chargé le programme informatique dans l'ordinateur, l'ordinateur réalise toutes les étapes du procédé de la revendication 5 lorsque le programme informatique est exécuté sur l'ordinateur.
